(19) Europäisches Patentamt
European Patent Office
Office européen des brevets

(11) EP 3 482 745 A1

(12) EUROPEAN PATENT APPLICATION

(43) Date of publication:
15.05.2019 Bulletin 2019/20

(51) Int Cl.:
A61K 9/16 (2006.01)     A61K 33/00 (2006.01)
A23L 5/00 (2016.01)

(21) Application number: 18204325.7

(22) Date of filing: 05.11.2018

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME
Designated Validation States:
KH MA MD TN

(30) Priority: 08.11.2017 IT 201700126965

(71) Applicant: DN 360 S.R.L.
56217 Pisa (PI) (IT)

(72) Inventors:
• Zambito, Ylenia
56121 Pisa (PI) (IT)
• Fabiano, Angela
56125 Pisa (PI) (IT)
• Piras, Anna Maria
57217 Livorno (LI) (IT)

(74) Representative: Fanfani, Stefano
Fanfani S.R.L.
Via Giovanni Bovio 19
50136 Firenze (IT)

(54) ORAL CONTROLLED-RELEASE CALCIUM COMPOUND AND METHOD FOR PREPARING IT

(57) A nutraceutical compound for gradual release of calcium in a human body, comprising zeolite powders enriched in calcium ions and a waxy excipient. A method for preparing said compound.

FIG. 5

## Description

### Technical Field

The present invention belongs to the sector of functional foods and, more specifically, it is a nutraceutical compound, i.e. a food substance which positively affects the physiological functions of a body, and a method for preparing it.

Specifically, the sector is that of formulations for controlled-release calcium, especially aiming at preventing osteoporosis and at treating its effects.

### Present status of the art

It is known that an insufficient intake of calcium contributes to decrease bony density and causes a premature bony decline, which an increased risk of fractures is associated with.

Lack of calcium, if lasting for a long time, plays a fundamental role in developing osteoporosis.

The latter is a systemic disease of the skeleton, whereby the skeletal mass diminishes without a relevant percentage alteration of the mineral component of the residual bone tissue, characterized by a reduced bone mass and a deterioration of the microarchitecture of the bone tissue, resulting in an increased fragility and predisposition to fractures, especially in the hip, spinal column, and wrist regions.

Calcium concentration in all the district of a body shall be monitored and the central mechanism for doing it is monitoring calcinemia, i.e. the concentration of calcium in blood. As a matter of fact, an appropriate level of calcium in blood is essential to provide the vital functions of a body such as, for instance, contraction of muscles, such as heart, coagulation of blood, conduction of nerve impulses. Bones are the reserve of calcium in a human body; the latter eliminates about 300 mg of calcium per day, through urines.

Regulation of calcinemia takes place thanks to the combined action of three hormones: parathormone, calcitonin, and vitamin D.

If the calcinemia level decreases, then the parathyroides secrete the parathormone, which causes calcium to get free from the bone and calcinemia to grow up, additionally the parathormone stimulates the kidney, thus fostering calcium recovery in the kidney tubules. Calcium continues being collected from the bone, as long as there is deficiency of calcium which results in a loss of bone tissue.

Conversely, the calcitonin comes into operation when the calcinemia level is high, and specifically makes calcium get lost with the urine and prevents bone reabsorption and release of calcium in blood.

Vitamin D stimulates the absorption of calcium in the intestine.

In order to prevent osteoporosis, it has been thought about increasing calcinemia by administering an appropriate amount of calcium, the value of which depends on patient's age. It is generally shared that an appropriate diet should contain a daily quantity of calcium equal to about 1000 mg in the first 8 years of life, 1600 mg in 9 to 17-year-old people, and 1100 mg in 18 to 30-year-old people.

The above specified quantities might vary on a case by case basis, and also the scientific literature does not fully agree on which the daily administration of calcium and vitamin D with the diet should be in the different phases of life. The variability of the data given by the literature and by different national and international organizations is related to the difficulties encountered in studying calcium requirements. As a matter of fact, there are no appropriate biochemical indicators that reflect the nutritional status of these elements; therefore, indirect indicators shall be used, such as evaluation of the bone mass, or even complex studies on calcium balance, clinical trials which better investigate the relationship existing between different calcium administrations and the mineral contents of bones.

At present the most common calcium integrators include calcium carbonate, calcium citrate, calcium chloride, and calcium gluconate salts.

Calcium integrators, with or without vitamin D, are beneficial or even essential whenever the daily diet is not capable of providing the necessary amount of calcium, for instance for those who do not eat dairy products. Patent application US 2005031708 (A1) discloses a pharmaceutical composition comprising a zeolite and calcium powder for use as a food integrator.

Calcium is absorbed by way of an active transport in the duodenum, as well as in the first part of the jejunum, by way of transporters whose synthesis is stimulated by vitamin D; also, it is absorbed by way of a passive-paracellular transport, all along the intestine, including, even though minimally, the colon. In a human body, paracellular calcium transport is not influenced by vitamin D and only depends on calcium solubility, chyme transit time through the intestine, and calcium paracellular permeability.

Active absorption becomes predominant when a meal is poor in calcium, whereas the fraction absorbed by way of passive transport increases as the contents of calcium in chyme increases.

An important limit of the known prior art formulations of calcium integrators is inherent to the fact that they do not allow for a gradual release of the calcium ion, but rather they exclusively depend on the solubility of the used salts in the gastrointestinal fluids.

Also, the therapies based on administering calcium salts showed a number of different contraindications caused by the onset of hypercalcinemia, the latter possibly causing nausea, vomiting, constipation, stomach pains, thirst, polyuria, polydipsia, hypertension, and vasomotor disorders; hypercalciuria, calcium lithiasis and serious kidney failure.

Calcium salts, such as phosphates and carboxylates, are little soluble in water and form non-amphoteric hydroxides. The calcium ions that form in the stomach by way of acid dissolution possibly precipitate in the form of hydroxides, when the partially digested chyme is transferred into the intestine and finds here a more and more alkaline environment, unless a formation of soluble complexes prevent calcium hydroxides from precipitating.

The process whereby calcium precipitates from supersaturated solutions, resulting from the pH of the chyme in the intestine being neutralized, might also be kinetically inhibited to increase bioavailability. However, such precipitation inhibitors or complexing agents shall be available in the intestine.

The reduced bioavailability of calcium, with its sanitary consequences, is apparently related to the calcium precipitating in this step of the digestion, but it might even be related to the absence of calcium complexing agents or calcium precipitation inhibitors.

Therefore, the calcium dissolved in the stomach tends to precipitate in intestine, thus causing side effects such as constipation, flatulent and abdominal swelling. Also, the present calcium integrators, if taken jointly with a diet rich in calcium, might cause hypercalcinemia, a pathology that is responsible for numerous side effects, among which the most severe ones affect the heart.

## Objects and summary of the invention

A first object of the present invention is thus to provide a compound capable of providing a gradual release of calcium during its gastrointestinal transit, in order to improve its bioavailability, thus reducing the daily dose necessary to a patient and thus also reducing the possible negative side effects related to calcium administration.

A second object of the invention is to provide a controlled release formulation, which is capable of releasing calcium in the intestine in such a way as to replace that which is absorbed, without contributing to increase concentration to a value greater than the maximum concentration of free calcium ions in the intestine. As a matter of fact, whereas calcium features a high solubility in the stomach, thanks to its pH being acid, it is practically insoluble in the intestine, where pH varies in the range from 6 to 8, depending on the section thereof. A further object of the present invention is thus to prevent calcium hydroxides from precipitating in intestine, thus also reducing side effects such as constipation, flatulence and abdominal swelling.

Advantageously this controlled release formulation also facilitates prevention of hypercalcinemia, a pathology that often turns up if the integrator is taken jointly with a diet rich in calcium.

In one particularly complete embodiment, the invention also achieves a further object of reducing the speed of gastrointestinal transit of that compound, so as to reduce or even zero the portion of calcium which remains trapped in the compound and is eliminated without producing any beneficial effects to the body.

Other objects and advantages of the invention will be apparent to those skilled in the art from reading the following text.

The above described objects are achieved by using a formulation which joins calcium-containing substances with mineral powders commonly called zeolites, and advantageously can this formulation be completed by using a waxy excipient.

Zeolites are hydrated aluminosilicates of alkaline and alkaline-earth metals, whose structures are crystalline or are arranged according to tetrahedra whose oxygen atoms are exchanged with adjacent tetrahedra.

Zeolites are a subject of investigations in different fields of pharmaceutical applications, for instance as vehicles for low molecular weight active principles, such as non-steroidal anti-inflammatory drugs (NSAID) or because of their ion exchanger characteristics and antimicrobic properties.

Thanks to the microporous structure of zeolites, the negative charges are possibly located not only on the surface of the particles, but also on the surface of the inner pores; said negative charges bind to the positive ions of calcium in given conditions.

A process to charge a zeolite powder with $Ca^{2+}$ ions comprises pouring the zeolite powder into water, for instance 5% by weight per unit volume, thus obtaining an aqueous suspension, which an equal volume of an aqueous quasi saturated $CaCl_2$ solution is subsequently added to. Then the thus obtained suspension is continuously stirred for a number of hours in standard temperature and pressure conditions; during such stirring, an ion exchange takes place on the surface of zeolite granules between the positive ions of the alkaline metals of zeolite, such as, for instance, magnesium ions, sodium ions, and potassium ions, with the calcium ions present in the quasi saturated $CaCl_2$ solution.

The very orderly microporous structure which characterizes zeolites makes the interface surface increase and foster the ion exchange process.

Particularly effective results are obtained by using zeolite of the clinoptilolite type; the results are even better if zeolite particles are used whose average diameter is less than 0.50 microns.

Subsequently the solution is centrifuged and the sediment resulting from centrifugation is used for preparing a nutraceutical compound, after being dried in a vacuum oven at a temperature of about 40°C.

The nutraceutical compound is finally obtained by adding a wax-based excipient to the dried sediment; the wax-based excipient is previously melted and then added to the dried sediment, while being stirred.

As discussed below, the waxy excipient contributes to delay calcium release, because the diffusion of the $Ca^{2+}$ ions from the zeolite is slowed down by the need for spreading along the aqueous channels that form in the granules whenever the latter get in contact with physiological fluids.

According to a practical and effective composition, the waxy excipient specifically comprises a mixture of glyceryl stearate and glyceryl palmitate, with a predominance of diesters; this excipient is known under the tradename Precirol®.

As a last operation, in order to prepare the final granules of the compound according to the present patent application, the mixture of dried sediment and waxy excipient is extruded to form granules whose optimum dimensions are in the order of one tenth of millimeter.

A number of different tests were performed in order to check the actual effectiveness of the compound according to the present patent application.

In order to identify the amount of calcium actually added to the zeolite, the $Ca^{2+}$ contents of the supernatant resulting from the centrifugation of the zeolite supplemented with a calcium solution was measured, after an appropriate dilution and by using the fura-2 indicator to determine the amount of $Ca^{2+}$ (EI) actually present in the zeolite, by using the following formula:

$$EI= [(Mt-Ms)/ Mt] \times 100$$

where Mt is the total mass of calcium used for the preparation and Ms is the mass found in the supernatant.

Starting from a volume of 10 ml of an aqueous 5% (w/v) zeolite solution, and adding a volume of 10 ml of a 0.7 M $CaCl_2$ solution in water, the amount of $Ca^{2+}$ (EI) found was 51.4±4,5%.

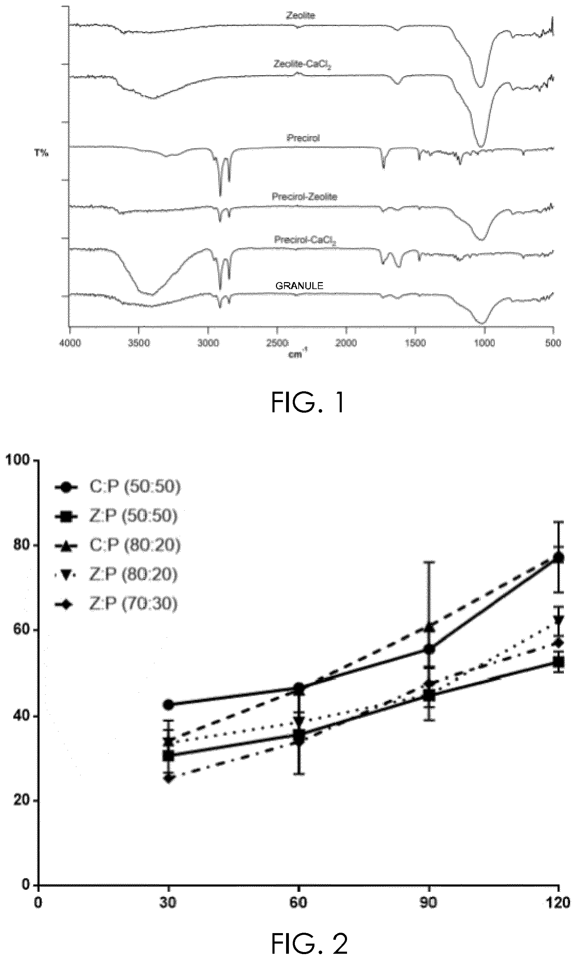

The components of the studied formulation were characterized by way of an attenuated total reflectance-Fourier transform infrared spectroscopy (ATR-FTIR) and the resulting spectra were compared to the spectrum obtained with 1.2 mm granules and a formulation containing 20% Precirol®, 61% $Ca^{2+}$ and 19% zeolite, here below in short referred to as Z:P (80:20) 1.2. The obtained data is shown in figure 1 and in Table 1.

It is also worth noting that zeolite, being an aluminum silicate, exhibits both the signals typical of Si-O-Si (Al) stretching (1027 and 795 $cm^{-1}$) and the H-O-H bands of the associated hydration water (3600-3400 and 1630 $cm^{-1}$).

The zeolite-$CaCl_2$ association does not result in any significant changes in the zeolite spectrum.

The characteristic bands of Precirol® are an asymmetrical and symmetrical stretching (2956-2848 $cm^{-1}$) and a bending (1471 and 1392 $cm^{-1}$) of the aliphatic chains, besides a stretching of the ester carbonyls (1730 $cm^{-1}$).

The Precirol®-$CaCl_2$ association does not result in any significant changes in the bands of the wax spectrum, whereas the Precirol®-zeolite association (in short Precirol®-Zeolite) is confirmed by the presence of the zeolite-characteristic bands.

In the spectrum of the final granules it is possible to identify the characteristic bands of the individual components: zeolite-$CaCl_2$ (silicate and associated hydrating water stretching) and Precirol® (aliphatic chain stretching and bending, ester carbonyl stretching).

A study was also made on release of $Ca^{2+}$ ions from the granulates of the compound according to the present patent application; the results are shown in figures 2, 3, and 4, a comment to which is found in the description of the tests performed.

## Brief description of the drawings

**Fig. 1** shows a diagram which compares the spectrum of the Z:P (80:20) 1.2 granulates with the spectra of the components of the formulation as obtained by ATR-FTIR spectroscopy. The spectra shown in the figure were obtained, from top down, from: zeolite, zeolite-$CaCl_2$, Precirol®, Precirol®-zeolite, Precirol®-$CaCl_2$ and Z:P (80:20) 1.2 granulates.

The subsequent figures show the percentage of $Ca^{2+}$ released over time, expressed in minutes, by granules immersed in elution means formed of simulated gastrointestinal fluids, such as:

- Simulated gastric fluid (SGF), formed of HCl 0.04 M, pH 1.2, made isotonic with NaCl (40 g of 1N HCl and 1 g of NaCl per 500 ml);
- Simulated jejunal fluid (SJF) pH 6.8;
- Simulated large intestinal environment (SLIE) pH 7.4. In particular, in order to measure the $Ca^{2+}$ release from the

granulates, a test was made wherein, at time t=0, the stirrer was immersed in the receiving phase (100 ml containing 100 mg of granulates to be tested) contained in a beaker and previously thermostatically controlled to 37 °C.

The fluids used for simulating the intestinal environment did not contain any ions that resulted in forming insoluble calcium salts; this is the reason why the experimental conditions used are to be considered those wherein a speed of release as high as possible is obtained.

The formulations of the granules are shown in Table 2 in terms of mass and dimensions.

**Fig. 2** shows the percentage of $Ca^{2+}$ released by 1.2mm granules immersed in a simulated gastric fluid (SGF). The four formulations are easily distinguishable from each other after 30 minutes, and are the following, from top down: C:P (50:50) 1.2, C:P (80:20) 1.2, Z:P (80:20) 1.2, Z:P (50:50) 1.2 and Z:P (70:30) 1.2.

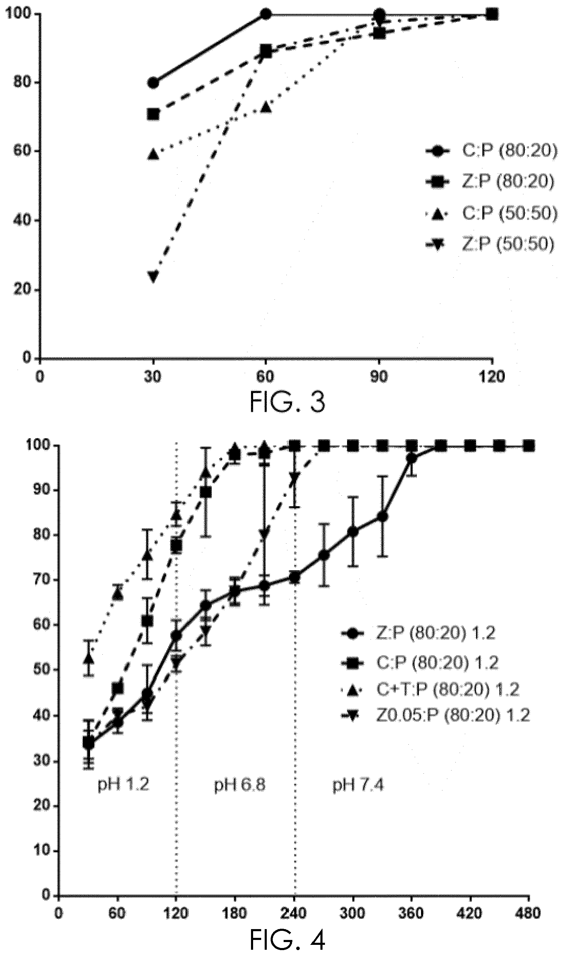

**Fig. 3** shows the percentage of $Ca^{2+}$ released by 0.9mm granules immersed in a simulated gastric fluid (SGF). The figure shows the release diagram of $Ca^{2+}$ of the formulations under investigation in a simulated gastric environment for 120 minutes. Also with reference to a 30 minutes time, the formulations tested the, from top down: C:P (80:20) 0.9, Z:P (80:20) 0.9, C:P (50:50) 0.9 and Z:P (50:50) 0.9.

It is inferred from the data shown in figures 2 and 3 that release is complete after a 2-hour elution in the case of 900-micron granules, whereas release is apparently delayed and influenced by zeolite in the case of 1200 micron granules.

Fig. 2 shows that whenever zeolite is present, in the first 2 hours release does not depend on the zeolite to Precirol® ration.

**Fig. 4** shows the percentage of $Ca^{2+}$ released by 1.2mm granules immersed, in the indicated sequence, in a simulated gastric fluid (SGF) for 120 minutes, in a simulated jejunal fluid (SJF) for 120 minutes, and then in a simulated large intestinal environment (SLIE).

The 20% Precirol®, 61% $Ca^{2+}$ and 19% zeolite formulation (Z:P (80:20) 1.2) was submitted to an intestinal digestion simulation (pH 6.8 and 7.4) together with two further formulations taken as references, both featuring the same percentage of Precirol®, namely 20%. One formulation includes calcium only as a further component, abbreviation C:P (80:20) 1.2, whereas the other contains talc (19%) and calcium (61%), abbreviation C+T:P (80:20) 1.2. Talc was selected as a reference because it features a chemical composition similar to that of zeolite. The Z0.05:P (80:20) 1.2 formulation includes zeolite particles having an average diameter less than 0.50 microns.

Fig. 4 shows how much zeolite is able to delay release of calcium as compared to the two reference formulations. As a matter of fact, whereas the zeolite-containing formulation results in a continuous and gradual release of calcium for about 6 hours, release of calcium is completed after 3 hours in the absence of zeolite.

On the basis of the release diagram obtained for the Z:P (80:20) 1.2 formulation, it can be speculated that release is controlled by diffusion of calcium ions internally to the aqueous channels that form inside the granules once they get in contact with physiological fluids. Such spreading is slowed down by the interaction of calcium ions with the negatively charged surface of zeolite. As a matter of fact, the granules consisting of calcium and Precirol® only (C:P (80:20) 1.2) were not able to adequately control calcium release.

The data also demonstrates that the particular monoporous molecular structure of zeolite is indispensable for controlling release, thanks to the increased specific surface made available upon interaction with calcium ions. As a matter of fact, the granules obtained with talc (C+T:P (80:20) 1.2) were not able to control release. If release were controlled by the calcium ions spreading through the aqueous channels of zeolite, then its speed would decrease over time, a circumstance that is not found in the diagram relevant to Z:P (80:20) 1.2, which, on the contrary, demonstrates a speeded up release after four hours as from the beginning of the test. This behavior can be related to a progressive break-up of granules as they empty out of calcium ion.

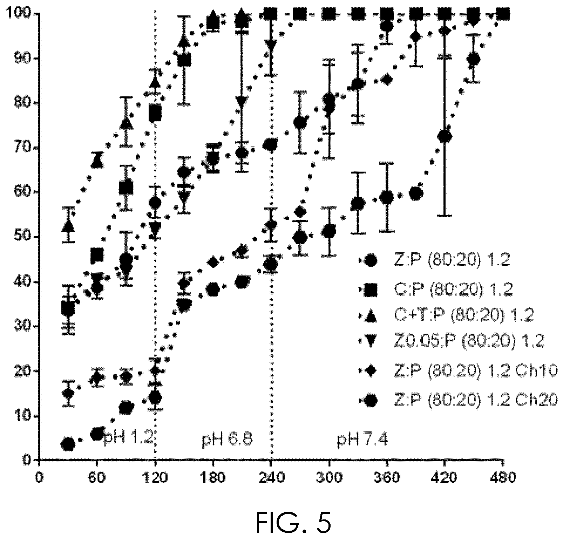

**Fig. 5** shows the same simulation parameters and conditions as those of figure 4, so as to allow for a comparison between the behavior of the Z:P (80:20) 1.2, C:P (80:20) 1.2 and C+T:P (80:20) 1.2 formulations and those of granulates obtained starting from the Z:P (80:20) 1.2 formulation, after a chitosan-based coating. The granules of the Z:P (80:20) 1.2 formulation were loaded in a coating pan and kept in a continuous movement and wetted with an aqueous 1% by weight chitosan solution, previously acidified to pH 6.4 with 1 molar hydrochloric acid. In the case here described, the application of the aqueous solution took place in several steps, up to coating granules with a volume of 10 mL or 20 mL of aqueous solution, so as to obtain the Z:P (80:20) 1.2 Ch10 formulation and the Z:P (80:20) 1.2 Ch20 formulation.

The figure shows a greater capability of the coated granules of controlling the spread of calcium ions in the aqueous channels that form inside the granules after they get in contact with physiological fluids. Such better control is apparently dependent on the coating degree achieved.

It follows that chitosan not only slows down the transit of granules through the gastrointestinal apparatus, but it also contributes to further slow down the speed of calcium release, thus enhancing the action performed by the waxy excipient.

## Detailed description of one embodiment of the invention

The following description is for explanatory purposes only. A known volume of a zeolite powder suspension in water is formed, featuring a zeolite powder to water ration, in grams/millimeter, equal to 5%.

It is here pointed out that said zeolite powder weight to water volume ratio is that which gave the best results during the experimental step, however said ratio might even substantially vary without precluding the effectiveness of the compound according to the present patent application. An equal volume of 10 ml of a quasi-saturated 0.7 M $CaCl_2$ solution in water is added, and the suspension is continuously stirred for 8 to 16 hours, generally 12 to 16 hours, usually 14 hours, at standard temperature and pressure (STP) conditions.

Particularly effective results are obtained by using zeolite powders of the clinoptilolite type, a natural zeolite whose empirical chemical formula is the following: $(Ca,K_2,Na_2,Mg)_4Al_8Si_{40}O_{96}\cdot24H_2O$.

The results are generally better if the average diameter of the zeolite particles used is less than 0.50 microns.

Then the suspension is centrifuged; good results are obtained for an overall volume of about 20 ml by centrifuging it at a speed of 2000 RPM for 10 minutes. After centrifuging and drying the sediment, the waxy excipient is heated up to make it melt; excellent results are obtained by heating the waxy excipient to a temperature of 75 °c, then the calcium-enriched zeolite is gradually added to the melted excipient, everything being continuously stirred.

According to a practical and effective composition, the waxy excipient comprises a mix of glyceryl stearate and glyceryl palmitate, with a predominance of diesters; such mixture is known under the tradename Precirol® ATO 5. Finally, the mixture is extruded, for instance by forcing it through a sieve or using an extruder. The different types of 0.9 to 1.2mm granules indicated in Table 2 were obtained by varying the opening of the mesh.

It was noted that calcium release is apparently not significantly influenced by the percentage of Precirol® present in the zeolite-based formulations; so, the Z:P (80:20) 1.2 formulation is apparently the most suitable one for implementing the rational of the present patent, because it contains a dose of calcium greater than the others, in addition to zeolite.

In a particularly complete embodiment, the nutraceutical compound according to the invention forms granules that are coated with a layer comprising a muco-adhesive polymer; good results were obtained by using chitosan. The presence of a muco-adhesive coating makes it possible to slow down the transit of granules through the gastrointestinal apparatus, thus preventing a transit speed greater than the calcium release one from leaving part of the calcium dose trapped in the granules without exploiting its action. Also, the chitosan-based muco-adhesive coating contributes, by itself, to further slow down the speed of calcium release, as shown in figure 5. According to a possible preparation of the granules used in the tests illustrated in figure 5, the granules of dusts immersed in a waxy excipient are treated with a previously acidified chitosan aqueous solution, in one or several subsequent steps; preferably are the granules kept in a continuous movement while being wetted with the aqueous solution.

**Table 1**

| | Hydrating $H_2O$ | $v_{as}$ C-H e $v_s$ C-H | | | vs C=O | Hydrating $H_2O$ | 8as C-H | 8s C-H | Silicate $v_{as}$ e $v_s$ Si-O | |
|---|---|---|---|---|---|---|---|---|---|---|
| Zeolite | 3600 | | | | | 1632 | | | 1027 | 795 |
| Zeolite $CaCl_2$ | 3400 | | | | | 1631 | | | 1027 | 795 |
| Precirol® | 3304 | 2955 | 2913 | 2848 | 1730 | | 1471 | 1392 | | |
| Precirol® Zeolite | 3600 | 2955 | 2915 | 2849 | 1734 | 1634 | 1471 | 1392 | 1027 | 795 |
| Precirol® $CaCl_2$ | 3403 | 2956 | 2914 | 2849 | 1734 | 1623 | 1471 | 1392 | | |
| Granule | 3400 | 2960 | 2916 | 2848 | 1735 | 1626 | 1470 | 1396 | 1020 | 798 |

**Table 2**

| Abbreviation | Precirol®, % | $Ca^{2+}$, % | Zeolite, % | Granule dimension, mm |
|---|---|---|---|---|
| Z:P (80:20) 0.9 | 20 | 61 | 19 | 0.9 |
| Z:P (70:30) 0.9 | 30 | 52.9 | 17.1 | 0.9 |

(continued)

| Abbreviation | Precirol®, % | Ca$^{2+}$, % | Zeolite, % | Granule dimension, mm |
|---|---|---|---|---|
| Z:P (50:50) 0.9 | 50 | 37.8 | 12.2 | 0.9 |
| C:P (80:20) 0.9 | 20 | 80 | - | 0.9 |
| C:P (70:30) 0.9 | 30 | 70 | - | 0.9 |
| C:P (50:50) 0.9 | 50 | 50 | - | 0.9 |
| Z:P (80:20) 1.2 | 20 | 61 | 19 | 1.2 |
| Z:P (70:30) 1.2 | 30 | 52.9 | 17.1 | 1.2 |
| Z:P (50:50) 1.2 | 50 | 37.8 | 12.2 | 1.2 |
| C:P (80:20) 1.2 | 20 | 80 | - | 1.2 |
| C:P (70:30) 1.2 | 30 | 70 | - | 1.2 |
| C:P (50:50) 1.2 | 50 | 50 | - | 1.2 |

**Claims**

1. An oral controlled-release calcium nutraceutical compound **characterized in that** it comprises Ca$^{2+}$ ion enriched zeolite powders, said zeolite powders being wound by at least one waxy excipient.

2. The compound according to claim 1, **characterized in that** said at least one waxy excipient comprises a mix of glyceryl stearate and glyceryl palmitate with a prevalence of diesters.

3. The compound according to any of the previous claims, **characterized in that** the average diameter of said zeolite powders is less than 50 microns.

4. The compound according to any of the previous claims, **characterized in that** the zeolite used is clinoptilolite.

5. The compound according to any of the previous claims **characterized in that** said zeolite powders wound by at least one waxy excipient form granules that are subsequently coated with a layer comprising a muco-adhesive polymer.

6. The compound according to the previous claim **characterized in that** said muco-adhesive polymer is chitosan.

7. A method for preparing the compound according to any of the previous claims, **characterized in that** it comprises the following steps:

   a) Forming a suspension of zeolite in water;
   b) Adding an identical volume of a quasi-saturated aqueous CaCl$_2$ solution to said suspension;
   c) Stirring said suspension at standard ambient temperature and pressure (STP);
   d) Centrifuging said suspension and drying the sediment thus obtained in a vacuum oven;
   e) Melting said at least one waxy excipient;
   f) Adding said dried sediment to said at least one melted waxy excipient and homogenizing the mixture;
   g) Extruding the mix to obtain granules.

8. The method according to the previous claim, **characterized in that** the zeolite aqueous suspension according to step a) is realized in a ratio of the weight of the zeolite powder, expressed in grams, to the volume of water, expressed in milliliters, equal to 5%.

9. The method according to any of claims 7 or 8, **characterized in that** said quasi-saturated aqueous CaCl$_2$ solution is a solution with a concentration of CaCl$_2$ equal to 0.7 M.

10. The method according to any of claims 7 thru 9 **characterized in that** it also comprises the following steps:

i) Wetting the granules obtained in said step g) of extruding the mixture, with an aqueous solution of a muco-adhesive polymer, in one or several successive steps.

**11.** The method according to the previous claim **characterized in that** during step i) of wetting the granules these are kept in a continuous movement.

**12.** The method according to claim 10 or 11 **characterized in that** said muco-adhesive polymer is chitosan.

**13.** The method according to claim 12 **characterized in that** said aqueous solution is a 1% by weight chitosan aqueous solution previously acidified with hydrochloric acid.

**14.** The nutraceutical compound according to any of claims 1 thru 6 for use as medicament and/or dietary supplement.

**15.** The nutraceutical compound according to any of claims 1 thru 6 for use in treatment of osteoporosis.

FIG. 1

FIG. 2

FIG. 3

Legend:
- C:P (80:20)
- Z:P (80:20)
- C:P (50:50)
- Z:P (50:50)

pH 1.2   pH 6.8   pH 7.4

Legend:
- Z:P (80:20) 1.2
- C:P (80:20) 1.2
- C+T:P (80:20) 1.2
- Z0.05:P (80:20) 1.2

FIG. 4

FIG. 5

Europäisches
Patentamt

European
Patent Office

Office européen
des brevets

# EUROPEAN SEARCH REPORT

Application Number

EP 18 20 4325

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | DE 10 2005 020467 A1 (KLINOMED INST FUER ANGEWANDTE [DE]) 19 October 2006 (2006-10-19) * paragraph [0019] * * paragraph [0023] * * paragraph [0027] - paragraph [0028] * * paragraph [0030] * ----- | 1-15 | INV. A61K9/16 A61K33/00 A23L5/00 |
| A | US 2005/031708 A1 (PORTNEY MICAH S [US]) 10 February 2005 (2005-02-10) * paragraph [0034] * * paragraph [0003] * * paragraph [0004] - paragraph [0005] * * paragraph [0014] * * paragraph [0015] * * paragraph [0030] * * paragraph [0033] * * paragraph [0035] * * paragraph [0038] * ----- | 1-15 | |

TECHNICAL FIELDS SEARCHED (IPC)

A61K
A23L

The present search report has been drawn up for all claims

| Place of search | Date of completion of the search | Examiner |
|---|---|---|
| The Hague | 25 March 2019 | Weiss, Marie-France |

EPO FORM 1503 03.82 (P04C01)

## ANNEX TO THE EUROPEAN SEARCH REPORT
## ON EUROPEAN PATENT APPLICATION NO.

EP 18 20 4325

This annex lists the patent family members relating to the patent documents cited in the above-mentioned European search report.
The members are as contained in the European Patent Office EDP file on
The European Patent Office is in no way liable for these particulars which are merely given for the purpose of information.

25-03-2019

| Patent document cited in search report | Publication date | Patent family member(s) | | Publication date |
|---|---|---|---|---|
| DE 102005020467 A1 | 19-10-2006 | DE 102005020467 A1 | | 19-10-2006 |
| | | WO 2006108414 A2 | | 19-10-2006 |
| US 2005031708 A1 | 10-02-2005 | CA 2534922 A1 | | 24-02-2005 |
| | | EP 1660044 A2 | | 31-05-2006 |
| | | US 2005031708 A1 | | 10-02-2005 |
| | | WO 2005016271 A2 | | 24-02-2005 |

For more details about this annex : see Official Journal of the European Patent Office, No. 12/82

**REFERENCES CITED IN THE DESCRIPTION**

**Patent documents cited in the description**

- US 2005031708 A1